# EUROPÄISCHE PATENTSCHRIFT

(11) **EP 3 515 399 B1**
(45) Veröffentlichungstag und Bekanntmachung des Hinweises auf die Patenterteilung: **31.03.2021**
(21) Anmeldenummer: 17781016.5
(22) Anmeldetag: 20.09.2017
(51) Int. Cl.: A61K 8/31, A61Q 9/00, A61Q 9/02, A61Q 9/04, A61Q 19/00, A61Q 19/02, A61Q 19/06, A61Q 19/08, A61K 8/891, A61K 8/893, A61K 8/895, A61K 8/898, A61K 8/19, A61K 31/282, A61K 31/80, A61P 17/00

(54) **KOSMETISCHE ODER PHARMAZEUTISCHE ZUSAMMENSETZUNG FÜR DIE HAUTSTRAFFUNG, HAUTFIXIERUNG UND FALTENREDUZIERUNG**
COSMETIC OR PHARMACEUTICAL COMPOSITION FOR SKIN TREATMENT, SKIN FIXATION AND WRINKLE REDUCTION
COMPOSITION COSMETIQUE OU PHARMACEUTIQUE POUR TRAITEMENT DE LA PEAU, FIXATION DE LA PEAU ET REDUCTION DES RIDES

(30) Priorität: 20.09.2016 DE 102016117728
(43) Veröffentlichungstag der Anmeldung: 31.07.2019
(73) Patentinhaber: Mayser Pharma AG, 6370 Stans (CH)
(72) Erfinder: BUSCH, Klaus-Uwe, 6004 Luzern (CH)
(74) Vertreter: WSL Patentanwälte Partnerschaft mbB
(86) Internationale Anmeldenummer: PCT/EP2017/073717
(87) Internationale Veröffentlichungsnummer: WO 2018/054941

(56) Entgegenhaltungen:
- EP-A1- 2 762 126
- DE-A1- 10 129 528
- DE-T2- 60 210 737
- DE-T2- 69 130 870
- DE-T2- 69 515 241

## Beschreibung

### GEGENSTAND DER ERFINDUNG

Die vorliegende Erfindung betrifft eine kosmetische oder pharmazeutische Zusammensetzung für die Hautstraffung, Hautfixierung und Faltenreduzierung sowie ein Verfahren zur kosmetischen oder pharmazeutischen Hautstraffung, Hautfixierung und Faltenreduzierung unter Verwendung der erfindungsgemäßen Zusammensetzung.

### HINTERGRUND DER ERFINDUNG

Die Haut ist ein Organ mit unterschiedlichen physiologischen Funktionen. Insbesondere dient sie dem Schutz gegenüber äußeren Einflüssen, wie z.B. extremen Temperaturen, Strahlung oder Mikroorganismen. Diese äußeren Faktoren führen über die Jahre zu einer Alterung der Haut, die durch weitere Faktoren wie Stress, Krankheiten oder chemische Reagenzien in Körper- und Schönheitspflegeprodukten verstärkt wird. Der Prozess der Hautalterung tritt verstärkt ab dem 30. Lebensjahr in Erscheinung und führt zur Bildung von Falten und Linien und einem Verlust von Elastizität und Spannkraft der Haut.

Neben der Alterung durch äußere Faktoren, gibt es auch eine intrinsische Hautalterung durch die genetisch gesteuerte verminderte Reagibilität der Hautzellen. Diese führt zu einem Abbau von Elastin und Kollagen im Gewebe. Außerdem reduziert sich der Wasser- und Fettgehalt der Haut signifikant, was dazu führt, dass das Hautbild relativ trocken erscheint und vermehrt Falten und Risse in der Haut entstehen.

### STAND DER TECHNIK

Um dem Prozess der Hautalterung entgegenzuwirken und/oder um die nach außen hin in Erscheinung tretenden Auswirkungen der Hautalterung zu kaschieren, wurden verschiedene kosmetische oder pharmazeutische Zusammensetzungen entwickelt.

Beispielsweise wird in DE 103 08 852 A1 als Mittel für die Verminderung der Faltenbildung der Haut eine wässrige Zusammensetzung vorgeschlagen, welche in Liposome oder Cerasome eingeschlossene Spasmolytika enthält.

In DE 10 2009 001 710 A1 wird eine kosmetische Zusammensetzung zur Reduktion von Hautfalten beschrieben, die in einem Wirkstoffträger aus Sphingolipiden und/oder Galactolipiden eine Wirkstoffkombination aus Hyaluronsäure und einem β-Glucan enthält.

EP 2 259 765 B1 offenbart ein kosmetisches Verfahren zur Glättung von Falten und feinen Linien mit Hilfe eines Interpolymers. Diese Zusammensetzung enthält wenigstens ein Polyurethan und wenigstens ein Copolymer von Vinylpyrrolidon und einem Siliconmonomer.

EP 2 382 961 A2 betrifft eine kosmetische Zusammensetzung zur optischen Kaschierung von Falten durch Siliconelastomere in Öl-in-Wasser-Emulsionen.

EP 1 299 080 A2 beschreibt Hautpflegezusammensetzungen, die Siliconelastomere enthalten. Diese sorgen für die therapeutische Regulierung von sichtbaren und/oder füllbaren Ungleichmäßigkeiten der Haut von Säugetieren einschließlich von Ungleichmäßigkeiten der Hauttextur und der Hautfarbe.

Langer et al. Nature Materials, 15, 911-918 (2016) offenbart die Anwendung von elastischen, tragbaren quervernetzten Polymerschichten auf der Haut, die die Eigenschaften normaler, junger Haut imitieren können. Die quervernetzten Polysiloxan-basierten Materialien können in ihrer Elastizität und Zugfestigkeit gezielt eingestellt werden. Als Polymerisationskatalysator dient eine Platinverbindung (Karstedt-Katalysator), die in einem zweiten Applikationsschritt aufgetragen wird, um die Polymerisation und Quervernetzung zu starten.

Die Wirkung der verschiedenen aus dem Stand der Technik bekannten Präparate ist häufig nicht so zufriedenstellend wie erwartet und oft auch nicht dazu geeignet, die Tiefe und Ausdehnung von bereits vorhandenen Hautfalten dauerhaft zu verringern. Herkömmliche kosmetische Zusammensetzungen, die Siliconverbindungen enthalten, sind außerdem sehr aufwendig zu applizieren und zudem problematisch bei Langzeitapplikation.

### AUFGABE

Demnach besteht ein Bedarf nach einer kosmetischen oder pharmazeutischen Zusammensetzung, die besser dazu geeignet ist, die Tiefe und Ausdehnung von Hautfalten zu reduzieren, als die aus dem Stand der Technik bekannten Präparate, und es war daher die Aufgabe der vorliegenden Erfindung eine entsprechend verbesserte Zusammensetzung bereitzustellen.

### BESCHREIBUNG DER ERFINDUNG

Diese Aufgabe wird erfindungsgemäß gelöst durch eine kosmetische oder pharmazeutische Zusammensetzung der eingangs beschriebenen Art, die für die topische Applikation auf die Haut geeignet ist und zusätzlich dadurch gekennzeichnet ist, dass sie wenigstens einen Polymervorläufer und wenigstens einen Polymerisationskatalysator enthält, in dessen Gegenwart der wenigstens eine Polymervorläufer unter Einwirkung von Licht polymerisiert.

Die erfindungsgemäße Zusammensetzung ist nach topischer Anwendung gemäß Anspruch 1 dazu geeignet, innerhalb weniger Minuten, vorzugsweise innerhalb weniger Sekunden, eine Polymerschicht auf der Haut mit außergewöhnlich angenehmem sensorischen Eindruck zu erzeugen. Diese Polymerschicht ist u.a. hervorragend dazu geeignet, die Haut zu straffen oder Falten und/oder kleine Linien an der behandelten Hautstelle zu kaschieren.

Die erfindungsgemäß erhaltene Polymerschicht ist auf der Haut tragbar, d.h. auch über mehrere Stunden (vorzugweise wenigstens 6 Stunden, wenigstens 12 oder wenigstens 24 Stunden) und Tage (bis zu 1 Tag, bis zu 3 Tage oder gar bis zu 7 Tage) der Anwendung auf der Haut ohne negative Hautreaktion verträglich. Vorzugsweise ist die erfindungsgemäß erhaltene Polymerschicht hierfür elastisch.

Der Begriff "kosmetische Zusammensetzung" ist hier so zu verstehen, dass hiermit eine für die kosmetische Anwendung geeignete Kombination von chemischen Stoffen gemeint ist. Der Begriff "pharmazeutische Zusammensetzung" ist hier so zu verstehen, dass hiermit eine für die pharmazeutische Anwendung geeignete Kombination von chemischen Stoffen gemeint ist.

Kosmetische angewandte Zusammensetzungen dienen allein der Körper- und Schönheitspflege, bzw. der Erhaltung, Wiederherstellung oder Verbesserung der Schönheit des menschlichen Körpers, im vorliegenden Fall insbesondere der Haut. Im Vergleich hierzu dienen pharmazeutische Zusammensetzungen primär der Verhinderung oder Heilung von Krankheitzuständen oder wenigstens der Beseitigung oder Linderung von deren Symptomen. Pharmazeutische Zusammensetzungen enthalten entsprechende Wirkstoffe und fallen - im Gegensatz zu rein kosmetischen Zusammensetzungen - unter das Arzneimittelrecht.

Die erfindungsgemäße Zusammensetzung ist für die topische Applikation auf die Haut bestimmt und dementsprechend auch hierfür geeignet. Unter "topischer Applikation auf die Haut" wird im Sinne der vorliegenden Erfindung das Auftragen der kosmetischen oder pharmazeutischen Zusammensetzung auf eine zu behandelnde Hautstelle verstanden.

Der Begriff "Haut" bezieht sich auf die Haut von Lebewesen, insbesondere die des Menschen, und umfasst im Zusammenhang mit der vorliegenden Erfindung sowohl die Hautschichten der Epidermis, Dermis und Subcutis, in Ihrer Gesamtheit oder jeweils für sich genommen, als auch die Hautanhangsgebilde, wie z.B. die Haare, Nägel, Talg- und Schweißdrüsen, in Ihrer Gesamtheit oder jeweils für sich genommen. Bei bevorzugten Ausführungsformen der Erfindung bezieht sich der Begriff nur auf die äußere Haut und/oder die äußeren Hautanhangsgebilde.

Die erfindungsgemäße kosmetische oder pharmazeutische Zusammensetzung enthält wenigstens einen Polymervorläufer, der in Gegenwart eines Polymerisationskatalysators unter Einwirkung von Licht polymerisiert.

Ein "Polymervorläufer" im Sinne der vorliegenden Erfindung ist eine Anzahl von wenigstens einem Monomer, das mindestens zwei in der katalysierten Polymerisationsreaktion reaktive Gruppen enthält und aus dem sich in der Polymerisationsreaktion ein Polymer bildet.

Ein "Polymerisationskatalysator" im Sinne der vorliegenden Erfindung ist ein chemischer Stoff, in dessen Gegenwart die Polymerisationsreaktion schneller abläuft und/oder bei geringeren Temperaturen und/oder unter Einwirkung von weniger Energiestrahlung als dies in Abwesenheit des Polymerisationskatalysators der Fall wäre. Durch die Gegenwart des Polymerisationskatalysators kann sich unter für die kosmetische Anwendung auf der Haut geeigneten Temperaturbedingungen ein Polymer innerhalb weniger Minuten oder bevorzugt innerhalb weniger Sekunden bilden.

Der Begriff "Polymerisation" ist hier so zu verstehen, dass damit der Prozess bezeichnet wird, bei dem ein Polymer ausgebildet wird. Im Sinne der vorliegenden Erfindung ist ein Polymer dann ausgebildet, wenn ein Polymerisationsgrad von mindestens 5, bevorzugt mindestens 10, noch bevorzugter mindestens 20, vorliegt.

Als Polymerisationsgrad wird die Anzahl der Monomereinheiten in einem Polymermolekül bezeichnet. Er ist identisch mit dem Quotienten der mittleren molaren Masse des Polymers und der molaren Masse seiner Wiederholeinheit.

In einer bevorzugten Ausführungsform handelt es sich bei dem gebildeten Polymer um ein quernetztes Polymer, also um ein Polymer, bei dem die Polymermoleküle wenigstens teilweise zu einem dreidimensionalen Netzwerk verknüpft sind.

Die Polymerisationsreaktion erfolgt erfindungsgemäß in Gegenwart eines Polymerisationskatalysators und unter gleichzeitiger Einwirkung von Licht.

Die Formulierung "in dessen Gegenwart" wird hier so verstanden, dass Polymervorläufer und Polymerisationskatalysator in der kosmetischen oder pharmazeutischen Zusammensetzung jedenfalls nach der erfolgten topischen Applikation in ein und demselben Gemisch auf der Haut vorliegen müssen. Dies kann entweder dadurch erreicht werden, dass Polymervorläufer und Polymerisationskatalysator im vorgefertigten Gemisch aufgetragen werden oder sequentiell in einem zeitlichen Abstand nacheinander.

Die Formulierung "unter Einwirkung von Licht" wird hier so verstanden, dass die vom Polymerisationskatalysator katalysierte Polymerisationreaktion ohne die Einwirkung von Licht entweder gar nicht oder jedenfalls nicht in der gewünschten Weise oder nicht mit der erforderlichen Geschwindigkeit abläuft. Für die Ausbildung des Polymers muss die kosmetische oder pharmazeutische Zusammensetzung nach deren topischer Applikation daher Licht ausgesetzt wird, wobei dieses Licht von einer künstlichen Strahlungsquelle oder von einer natürlichen Strahlungsquelle (z.B. Sonnenlicht) stammen kann.

Unter dem Begriff "Licht" wird hier Strahlung im Wellenlängenbereich von 200 nm bis 800 nm, vorzugsweise von 380 nm bis 800 nm, besonders bevorzugt 550 nm bis 780 nm, verstanden. Strahlung im Bereich von 200 nm bis 400 nm sorgt für eine schnellere Bildung der Polymerschicht, wohingegen Strahlung im Bereich von 380 nm bis 800 nm zwar mit einer etwas langsameren Polymerisationsreaktion verbunden ist, dafür aber etwas schonender für den Patienten ist.

Die Zeitangabe der Polymerisation "innerhalb von wenigen Minuten" umfasst vorzugsweise einen Zeitraum von weniger als 15 Minuten, noch bevorzugter weniger als 10 Minuten. Bei bestimmten Ausführungsformen erfolgt die Polymerisation innerhalb von 1 bis 10 Minuten, noch bevorzugter innerhalb von 1 bis 5 Minuten. Die Zeitangabe der Polymerisation "innerhalb von wenigen Sekunden" umfasst vorzugsweise einen Zeitraum von weniger als 60 Sekunden, noch bevorzugter weniger als 30 Sekunden. Bei bestimmten Ausführungsformen erfolgt die Polymerisation innerhalb von 5 bis 60 Sekunden, noch bevorzugter innerhalb von 5 bis 30 Sekunden.

In einer erfindungsgemäßen kosmetischen oder pharmazeutischen Zusammensetzung kann der Polymervorläufer ein Siloxan sein. In einer bevorzugten Ausführungsform ist das Siloxan ein polyethermodifiziertes Siloxan. Der Vorteil von polyethermodifizierten Siloxanen ist eine Verbesserung des Verlaufs und der Oberflächenglätte der Zusammensetzung.

Eine bevorzugte Ausführungsform der erfindungsgemäßen kosmetischen oder pharmazeutischen Zusammensetzung enthält als Polymervorläufer entweder
a) eine Kombination von
   i) mindestens einem Polyorganohydrosiloxan mit reaktiven Silangruppen zur Hydrosilylierung mit folgender Struktur: 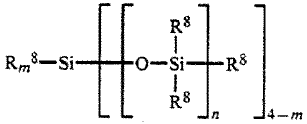 wobei R⁸ unabhängige Reste darstellen, ausgewählt aus einer Gruppe bestehend aus Alkylgruppen, Cycloalkylgruppen, Epoxidgruppen, Isocyanatgruppen, Aminogruppen, Alkoholgruppen, Ethergruppen, Estergruppen, Phenylgruppen und Wasserstoff, vorausgesetzt, dass mindestens zwei aber nicht mehr als die Hälfte aller R⁸-Gruppen des Siloxans Wasserstoffe sind und, wobei m 0, 1, 2 oder 3 und n eine Zahl mit einem Wert zwischen 1 und 3000 ist,
      und
   (ii) mindestens einem Polyorganosiloxan, das ungesättigte Gruppen zur Hydrosilylierung enthält, mit folgender Struktur: 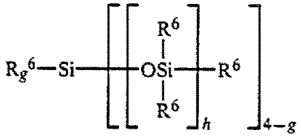 wobei R⁶ unabhängige Reste darstellen, ausgewählt aus einer Gruppe bestehend aus nicht-halogenierten oder halogenierten ethylenylisch ungesättigten Gruppen, nicht halogenierten oder halogenierten Alkylgruppen, nicht halogenierten oder halogenierten Cycloalkylgruppen, Epoxidgruppen, Isocyanatgruppen, Aminogruppen, Alkoholgruppen, Ethergruppen, Estergruppen, Phenylgruppen, vorausgesetzt, dass mindestens 70% aller R⁶ Gruppen Vinylgruppen oder andere Alkenylgruppen enthalten und wobei h eine Zahl mit einem Wert zwischen 1 und 3000 und g 0, 1, 2, oder 3 ist,
   oder
b) mindestens ein Polyorganohydrosiloxan mit folgender Struktur, das ungesättigte Funktionen als auch Silangruppen zur Hydrosylilierung in einem Molekül enthält: 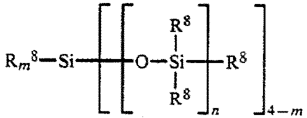 wobei R⁸ unabhängige Reste darstellen, ausgewählt aus einer Gruppe bestehend aus Alkylgruppen, Cycloalkylgruppen, Phenylgruppen, Epoxidgruppen, Isocyanatgruppen, Aminogruppen, Alkoholgruppen, Ethergruppen, Estergruppen Wasserstoff und nicht-halogenierten oder halogenierten ethylenylisch ungesättigten Gruppen, vorausgesetzt, dass mindestens zwei aber nicht mehr als die Hälfte aller R⁸-Gruppen des Siloxans Wasserstoffe sind und weiterhin vorausgesetzt, dass mindestens zwei der R⁸-Gruppen Vinylgruppen oder Alkenylgruppen enthalten, wobei m 0, 1, 2 oder 3 und n eine Zahl mit einem Wert zwischen 1 und 3000 ist.

Eine bevorzugte Ausführungsform der erfindungsgemäßen kosmetischen oder pharmazeutischen Zusammensetzung gemäß der oben genannten Alternative a) enthält als Polymervorläufer eine Kombination von i) und ii), wobei mindestens 70% aller R⁶-Gruppen Vinylgruppen oder andere Alkenylgruppen sind.

Eine bevorzugte Ausführungsform der erfindungsgemäßen kosmetischen oder pharmazeutischen Zusammensetzung gemäß der oben genannten Alternative b) enthält als Polymervorläufer ein Polyorganohydrosiloxan, wobei mindestens zwei der R⁸-Gruppen Vinylgruppen oder Alkenylgruppen sind.

Die erfindungsgemäße Zusammensetzung enthält einen Polymerisationskatalysator, also einen chemischen Stoff, in dessen Gegenwart die Polymerisation schneller abläuft und/oder bei geringeren Temperaturen und/oder unter Einwirkung von weniger Energiestrahlung als dies in Abwesenheit des Polymerisationskatalysators der Fall wäre.

Bei bestimmten Ausführungsformen der Erfindung ist der Polymerisationskatalysator eine Verbindung, die unter die allgemeine Formel (II) fällt, 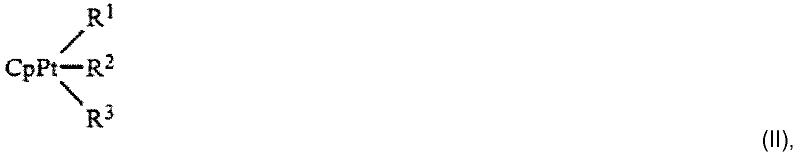 wobei Cp eine Cyclopentadienylgruppe darstellt, die η-gebunden an das Platinatom vorliegt, wobei die Cyclopentadienylgruppe wahlweise ein- oder mehrfach substituiert ist mit Resten, die nicht bei der Hydrosilylierungsreaktion stören, und jeder der Reste R¹, R² und R³ eine aliphatische oder aromatische Gruppe mit 1-18 Kohlenstoffatomen darstellt, wobei R¹, R² und R³ σ-gebunden an das Platinatom vorliegen, und wobei die Zusammensetzung zusätzlich wenigstens einen Photosensibilisator enthält, der dazu geeignet ist, die absorbierte Lichtenergie auf den Polymerisationskatalysator zu übertragen.

Bei bestimmten Ausführungsformen der Erfindung muss zusätzlich zu dem Polymerisationskatalysator ein Photosensibilisator vorgesehen sein, der Licht im hier angewandten Bereich absorbiert und die so absorbierte Energie für Zwecke der Katalyse der Polymerisationreaktion in geeigneter Form bereitstellen kann.

Für die vorliegende Erfindung geeignete Photosensibilisatoren sind solche Verbindungen, die Licht innerhalb des erfindungsgemäß angewandten Bereichs absorbieren können und auf den PlatinKomplex Energie übertragen können. Vorzugsweise werden entsprechende Photosensibilisatoren ausgewählt unter polycyclischen aromatischen Verbindungen, vorzugsweise aromatischen Verbindungen mit 2 bis 5 Ringen, Thioxanthonen, 2-Chlorthioxanthonen, 2-Isopropylthioxanthonen und aromatischen Verbindungen mit mindestens einem Ketonchromophor.

Die Verbindungen des Photosensibilisators können mit einem beliebigen Substituenten substituiert sein, der die Fähigkeiten der Verbindung des Photosensibilisators oder des Hydrosilylierungskatalysators zum Absorbieren von Licht und übertragen von Energie nicht beeinträchtigt. Beispiele für typische Substituenten sind: Alkyl-, Alkoxy-, Aryl-, Aryloxy-, Aralkyl-, Alkaryl- und Halogen-Substituenten.

Als spezielle Beispiele für polycyclische aromatische Photosensibilisatoren, die für die Erfindung geeignet sind, können Anthracen, 9-Vinylanthracen, 9,10-Dimethylanthracen, 9,10-Dichloranthracen, 9,10-Dibromanthracen, 9,10-Diethylanthracen, 9,10-Diethoxyanthracen, 2-Ethyl-9,10-dimethylanthracen, Naphthacen, Pentacen, Benz-[a]arithracen, 7,12-Dimethylbenz[a]anthracen und Azulen genannt werden.

Als spezielle Beispiele für aromatische Ketone, die als Photosensibilisator für die vorliegende Erfindung geeignet sind, können 2-Chlorthioxanthon, 2-Isopropylthioxanthon, Thioxanthon, Anthrachinon, Benzophenon, 1-Chloranthrachinon und Bianthron genannt werden.

Bei einer bestimmten Ausführungsform der zuvor beschriebenen Variante der vorliegenden Erfindung, bei der ein Photosensibilisator zum Einsatz kommt, wird ein Platinkomplex, mit einer η-gebundenen Cyclopentadienylgruppe in Verbindung mit einem Photosensibilisator, für die Katalyse der Polymerisation genutzt. Der Hydrosilylierungsprozess wird durch sichtbares Licht induziert, wobei der Photosensibilisator das sichtbare Licht absorbiert und die Energie auf den Platinkomplex überträgt.

Bei bestimmten Ausführungsformen der Erfindung muss kein zusätzlicher Photosensibilisator vorgesehen sein, dann nämlich wenn der Polymerisationskatalysator selbst Licht im hier angewandten Bereich absorbieren und die so absorbierte Energie für Zwecke der Katalyse der Polymerisationreaktion in geeigneter Form bereitstellen kann.

Ein Beispiel für einen für die vorliegende Erfindung geeigneten Polymerisationkatalysator, der selbst Licht für Zwecke der Bereitstellung der absorbierten Energie für die Katalyse der Polymerisationreaktion absorbieren kann, ist die η-Cyclopentadienyl-tri(σ-hydrocarbyl)platinverbindung gemäß Formel (I), wie offenbart in US 8,088,878 B2, wobei diese durch eine Alkyl Gruppe an eine hydrolysierbare Silylgroup geknüpft ist, wie angegeben in Anspruch 4 dieser Anmeldung. Dieser Katalysator kann durch sichtbares Licht angeregt werden, um Hydrosilylierungsreaktionen zu katalysieren.

Während bei anderen Katalysatoren auf Platinbasis die Gefahr besteht, dass die relativ kleinen, unpolaren Moleküle die Hautbarriere passieren, erfolgt bei der Reaktion mit diesem Katalysator eine Einbindung in die Silikonmatrix ohne die Ligandenstruktur wesentlich zu verändern oder die Katalysatoraktivität negativ zu beeinträchtigen. Hierdurch wird ebenso der Dampfdruck der Platinverbindung stark abgesenkt, was die Aufnahme als Feinpartikel über die Atemwege und eine damit verbundene mögliche toxische Wirkung stark reduziert.

Die erfindungsgemäße kosmetische oder pharmazeutische Zusammensetzung enthält wenigstens einen Polymervorläufer und einen Polymerisationskatalysator zur Polymerisation des Polymervorläufers unter Einwirkung von Licht.

Vorzugsweise besteht die kosmetische oder pharmazeutische Zusammensetzung der Erfindung aus einem Stoffgemisch, das sowohl den Polymervorläufer als auch den Polymerisationskatalysator enthält, sodass diese beiden Bestandteile gleichzeitig in Form eines vorgefertigten Gemisches auf die Haut aufgetragen werden. Bei einer speziellen Ausführungsform kann die kosmetische oder pharmazeutische Zusammensetzung jedoch auch aus zwei oder mehreren separat vorgehaltenen Komponenten bestehen, in denen der Polymervorläufer und der Polymerisationskatalysator getrennt voneinander enthalten sind. Auf diese Weise werden der Polymervorläufer und der Polymerisationskatalysator entweder erst kurz vor der Applikation gemischt oder sogar nacheinander auf die Haut aufgetragen und so erst *in situ,* d.h. unmittelbar vor der Applikation oder sogar erst während der Applikation miteinander in Kontakt gebracht und vermischt.

Bei bestimmten Ausführungsformen kann die kosmetische oder pharmazeutische Zusammensetzung zusätzlich Emulgatoren, Alterungsschutzmittel, eine Partikelphase, eine Öl-Wasser-Trägerphase, Antioxidantien, Salze oder Kombinationen hiervon enthalten.

Die erfindungsgemäße kosmetische oder pharmazeutische Zusammensetzung kann in Form eines Gels, einer Creme, einer Salbe, eines Sprays, einer Lotion, einer Öl-Wasser-Emulsion, eines Hydrogel, eines Balsam, einer Lösung oder einer Suspension vorliegen.

Bei einer bestimmten Ausführungsform der Erfindung liegt die kosmetische oder pharmazeutische Zusammensetzung in Form einer Öl-in-Wasser Emulsion vor, wobei das Gesamtgewicht der Zusammensetzung 5-70 Gew.-% Ölphase enthält. Die Ölphase kann ein oder mehrere Siliconöle enthalten, die ausgewählt sind aus cyclischen oder linearen Dialkyl-, Alkylaryl- und Arylsiloxanen. Beispiele sind Cyclopentasiloxan, Dimethylpolysiloxan, Phenylphenylpolysiloxan und Methylphenylpolysiloxan

Die Ölphase kann auch ein oder mehrere Esteröle enthalten, besonders bevorzugt sind Esteröle der Benzoesäure mit linearen oder verzweigten C₅-₂₅ Alkanolen.

Weitere kosmetische Öle, die in der Ölphase enthalten sein können, sind flüssige Paraffinöle, Isoparaffinöle und synthetische Kohlenwasserstoffe wie beispielsweise Di-n-Alkylether mit einer Kettenlänge C5-25.

Weitere erfindungsgemäße Öle, die in der kosmetischen oder pharmazeutischen Zusammensetzung enthalten sein können, sind lineare oder verzweigte, gesättigte oder ungesättigte Fettalkohole mit 6-30 Kohlenstoffatomen oder pflanzliche Öle wie beispielsweise Olivenöl, Sojaöl, Sesamöl, Haselnussöl, Maisöl oder Jojobaöl.

Die erfindungsgemäße kosmetische oder pharmazeutische Zusammensetzung kann weiterhin, bezogen auf ihr Gesamtgewicht 10-40 Gew.-% einer dispergierten Partikelphase enthalten. Die Partikel der Partikelphase weisen einen zahlenmittleren Partikeldurchmesser von 1-100 µm, bevorzugt 2- 50 µm, besonders bevorzugt 5-25 µm auf. Wesentlicher Bestandteil dieser dispergierten Partikelphase ist ein Siliconelastomer, das in der wässrigen Phase oder in der Emulsion dispergiert vorliegt und nicht die Ölphase verdickt. Derartige Siliconelastomere werden in EP 2 382 961 A2 näher beschrieben und verbessern wesentlich das Faltenkaschiervermögen der Zusammensetzung gemäß der vorliegenden Erfindung.

Weitere spezielle Ausführungsformen der Erfindung sind dadurch gekennzeichnet, dass die Partikelphase neben Siliconpartikeln weitere Partikel enthält, die aus folgenden Partikeln ausgewählt sind: Stärke und Stärkederivate, Cellulosepulver, Polymerpulver aus Polyolefinen, Polycarbonaten, Polyurethanen, Polyamiden, Polyester, Polystyrolen, Polyacrylaten, Polymethacrylaten, (Meth)acrylat- oder (Meth)acrylat-Vinyliden-Copolymeren, Teflon oder Kombinationen dieser.

In einer weiteren erfindungsgemäßen Ausführungsform enthält die Zusammensetzung 40-85 Gew.-%, bevorzugt 50-75 Gew.-%, besonders bevorzugt 51-60 Gew.-% an wässriger Phase, bezogen auf das Gesamtgewicht der erfindungsgemäßen Zusammensetzung.

Die wässrige Phase kann neben Wasser Di- oder Polyole wie beispielsweise 1,2 Propylenglycol, Glycerin, Butylenglykol, 1,6-Hexandiol oder Polyethylenglycole sowie Kombinationen dieser enthalten. Die wässrige Phase kann zudem anorganische Salze wie beispielsweise Natriumchlorid und/oder Basen wie beispielsweise Natriumhydroxid enthalten.

Die erfindungsgemäße Zusammensetzung kann mindestens einen Emulgator enthalten, wobei insgesamt 0,01 bis 1 Gew.-% Emulgator(en), bevorzugt 0,1 bis 0,5 Gew.-%, besonders bevorzugt 0,2 bis 0,4 Gew.-% in Bezug auf das Gesamtgewicht der erfindungsgemäßen Zusammensetzung, enthalten sind.

Bevorzugte Emulgatoren sind Lecithine, Silicon-Copolyole mit Ethylenoxid Einheiten, Alkylmono-und -oligoglycoside mit 8 bis 22 Kohlenstoffatomen im Alkyrest und deren ethoxylierten Analoga, ethoxylierte Sterine, Partialester von Polyglycerinen mit 2 bis 10 Glycerineinheiten und mit 1 bis 4 gesättigten oder ungesättigten, linearen oder verzweigten oder cyclischen, gegebenenfalls hydroxylierten C₈-C₃₀ Fettsäureestern verestert, sowie Kombinationen der genannten Substanzen.

In einer Ausführungsform enthält die kosmetische oder pharmazeutische Zusammensetzung Alterungsschutzmittel wie beispielsweise Mittel zur Stimulierung der Collagenproduktion, Mittel zur Stimulierung der Glykosaminoglykansynthese und der Proteoglykansynthese, Mittel zur Stimulierung der Integrinsynthese in den Fibroplasten, Mittel zur Stimulierung der Fructosamine-3-kinase- und der dazugehörigen Proteinexpression, Mittel zur Stimulierung der Tensinexpression, Mittel, die die Produktion von Wachstumsfaktoren fördern, Mittel, die die Proliferation der Fibroplasten anregen, Mittel, die die Proliferation von Keratinzyten anregen, Antioxidantien, Radikalfänger und Kombinationen der genannten Verbindungen. Beispiele solcher Verbindungen sind: Pflanzenextrakte von Butea Frondosa, Magnolia Champaca, Vanilla Planifolia, Cedrus Atlantica, Canarium Commune, Zingiber Cassumunar Roxb., Chondrus Crispus, Thermus Thermophilus, Pisum Sativum, Centella Asiatica, Scenedesmus, Moringa Pterygosperma, Hamamelis, Castanea Sativa, Hibiscus Sabdriffa, Polyanthes Tuberosa, Argania Spinosa, Aloe Vera, Narcissus Tarzetta, oder Liquorice,Pflanzenöl von Citrus Aurantium (Neroli), α-Hydroxysäuren, insbesondere Glykol-, Milch- und cyclische Säuren, und deren Estern, β-Hydroxysäuren, insbesondere Salicylsäure und deren Derivate, Hydrolysate von Pflanzenproteinen, acylierte Oligopeptide, Hefeextrakte, insbesondere Extrakte von Saccharomyces Cerevisiae, Extrakte von Algen, insbesondere von Braunalgen, Vitamine und deren Derivate, insbesondere Retinylpalmitate, Ascorbinsäure, Ascorbylglucoside, Magnesium-oder Natriumascorbylphosphat, Ascorbylpalmitat, Ascorbyltetraisopalmitat, Ascorbylsorbat, Tocopherol, Tocopherylacetat und Tocopherylsorbate, alkylierte oder phosphorylierte Ester der Ascorbinsäure, oder von Tocopherol oder deren Ester, Komplexbilder, insbesondere EDTA Salze, pH Regulatoren, Konservierungsmittel, insbesondere Parabene und Phenoxyethanol und Kombinationen hiervon.

Erfindungsgemäß kann die Polymerisation des Materials entweder in einem rein lichtinduzierten und -getriebenen Prozess erfolgen oder durch Dualpolymerisation. Unter Dualpolymerisation ist dabei die Initiation des Polymerisationsprozesses durch Licht, mit anschließender chemischer Polymerisation, oder in umgekehrter Reihenfolge zu verstehen. Der Mechanismus der chemischen Polymerisation kann dabei unter einer Kondensationsreaktionspolymerisation, einer Additionsreaktionspolymerisation, einer Ringöffnungsreaktionspolymerisation, und/oder einer katalytischen Polymerisation ausgewählt sein.

Erfindungsgemäß kann die Zusammensetzung zur Faltenreduzierung eingesetzt werden, indem die an der behandelten Stelle applizierte Zusammensetzung in die Hautfalten eindringt, diese z.T. auffüllt und nach der Polymerisation in einer vorteilhaften Position fixiert, um die vorhandenen Hautfalten auf diese Weise optisch zu kaschieren.

Erfindungsgemäß kann die Zusammensetzung auch allgemein zur Straffung oder Fixierung der Haut in einer gewünschten Position verwendet werden. Hierzu wird die Haut manuell in eine gewünschte Position gebracht und die Zusammensetzung appliziert. Alternativ wird zunächst die Zusammensetzung appliziert und dann die Haut manuell in eine gewünschte Position gebracht. Nach Polymerisation der Zusammensetzung verbleibt die Haut in der gewünschten Position. Dies kann beispielsweise gewünscht sein, um die Haut an der behandelten Stelle aus ästhetischen Gründen zu straffen und u.a. so an dieser Stelle an sich vorhandene Falten zu verringern. Es kann aber auch gewünscht sein, die Haut z.B. über Nacht in einer möglichst straffen, faltenfreien Form zu halten, um der Faltenbildung durch unbewusste Mimik oder mechanischen Einfluss (z.B. Kopfkissen) vorzubeugen. Es kann auf diese Weise sogar einzelnen Gesichtspartien eine gegenüber der natürlichen Gestalt andere Form gegeben werden.

Zur Verstärkung des vorgenannten Effekts kann die Zusammensetzung zusätzlich Stoffe mit klebendem Effekt enthalten. Beispiele hierfür sind: selbstklebende Polyurethane, Epoxidharze, Naturharze, Polyesterharze, ABS-Harze, Aminoplaste, Phenolharze, Poly(methyl)methacrylate, Polycyanoacrylate.

Die erfindungsgemäße Zusammensetzung kann auch großflächig und mit größerer Schichtdicke zur Körperformgebung angewandt werden, z.B. können mit der erfindungsgemäßen Zusammensetzung Schichten ausgebildet werden, welche die Funktion eines Büstenhalters übernehmen können.

Die erfindungsgemäße Zusammensetzung kann auch großflächig und mit größerer Schichtdicke angewandt werden, um eine Schutzschicht gegenüber Umwelteinflüssen auszubilden. Z.B. kann mit der erfindungsgemäßen Zusammensetzung eine durchgängige Schutzschicht auf der Haut der Hände ausgebildet werden, welche die Funktion von maßgeschneiderten Handschuhen übernehmen kann.

Erfindungsgemäß kann die Zusammensetzung auch zur Applikation und Fixierung von Schminkstoffen genutzt werden. Hierzu kann die Zusammensetzung zusätzliche dekorative Pigmente oder Farbstoffe enthalten. Beispiele hierfür sind Metalleffektpigmente, Perlglanzpigmente, Leuchtpigmente, natürliche organische Pigmente, synthetische organische Pigmente. Pigment- oder Farbstoffhaltige Schichten können auch zur Abdeckung von unschönen Narben oder von Hautstellen mit Pigmentstörung dienen.

Erfindungsgemäß kann die Zusammensetzung auch als Flüssigpflaster oder vorgefertigtes hoch elastisches Pflaster genutzt werden. Durch die Volumenkontraktion während der Polymerisation wird eine Kraft auf die Wundränder ausgeübt und diese näher zusammen gebracht. Dies sorgt für eine verbesserte Wundheilung. Anwendung können solche Pflaster als chirurgische Wundverschlüsse, Blasenpflaster, Wirkstoffpflaster oder auch als Sprühpflaster finden.

Erfindungsgemäß kann die Zusammensetzung auch zur Formgebung und/oder Fixierung von Körperhaaren und/oder künstlichen Haaren genutzt werden. Direkt auf das Eigen-, Fremd- oder Kunsthaar aufgebrachte Schichten der erfindungsgemäßen Zusammensetzung können zur Formgebung, und wahlweise auch zu Farbgebung, eingesetzt werden. In auf die eigene Haut oder in auf die eigenen Haare aufgebrachte Schichten der erfindungsgemäßen Zusammensetzung können aber auch natürliches Fremdhaar oder künstliche Haare eingebettet werden, um dieses Haar auf der eigenen Haut oder an den eigenen Haaren zu befestigen.

Weiterhin kann die erfindungsgemäße Zusammensetzung zur Entfernung von Haaren genutzt werden. Hierzu wird die Zusammensetzung auf eine Hautstelle appliziert und im Anschluss an die Polymerisation die entstandene Schicht abgezogen werden. Zur Verstärkung dieses Effekts können oben genannte Zusatzstoffe mit klebenden Eigenschaften genutzt werden.

Erfindungsgemäß kann die Zusammensetzung in Verbindung mit einem Wirkstoff zur Herstellung einer pharmazeutischen Zubereitung verwendet werden, die für Behandlung von Hautalterung und mit Hautalterung assoziierten Symptomen und zur Steigerung der Langlebigkeit von Hautzellen geeignet ist. Beispielsweise kann eine pharmazeutische Zusammensetzung im Sinne der vorliegenden Erfindung zusätzlich einen Sirtuin-Aktivator, wie z.B. Quercitin, Piceatannol, Resveratrol oder Isonicotinamid enthalten.

Die Formulierungen können alle Hilfs- und Zusatzstoffe, die üblicherweise bei kosmetischen oder pharmazeutischen Präparaten Anwendung finden, enthalten. Insbesondere fallen unter den Begriff "Hilfsstoff" im Zusammenhang mit der vorliegenden Erfindung solche Zusatzstoffe, die auf die physikalischen Eigenschaften der Wirkstoffe und/oder Vesikel und deren Stabilität einwirken und/oder der Konservierung der Zusammensetzung oder Wirkstoffzusammensetzung dienen. Beispiele für solche Hilfsstoffe sind Öle, Alkohole, Polyole, Gelbildner, Puffer, Konservierungsmittel, Bakterizide und Keimhemmer, Komplexbildner, Verdicker oder Konsistenzgeber.

Selbstverständlich handelt es sich bei allen Komponenten der erfindungsgemäßen Zusammensetzungen, Wirkstoffzusammensetzungen und Formulierungen um pharmazeutisch, kosmetisch oder dermatologisch verträgliche Stoffe. Ein Stoff ist im Sinne dieser Erfindung pharmazeutisch, kosmetisch oder dermatologisch verträglich, wenn er nicht toxisch ist und bei der Mehrzahl der potentiellen Anwender topisch anwendbar ist, ohne dass es bei dem Anwender spontan oder nach einer Weile zu einer ungewünschten physiologischen Reaktion, wie z.B. einer Rötung oder der Ausbildung eines Juckreizes kommt.

Die erfindungsgemäße Zusammensetzung kann entweder rein kosmetisch (nicht-therapeutisch), rein pharmazeutisch (therapeutisch) oder kombiniert (kosmetisch/pharmazeutisch) verwendet werden. Vorzugsweise erfolgt die Verwendung als rein kosmetische Zusammensetzung zu einem der vorgenannten Zwecke, z.B. zur optischen Kaschierung von Falten und Rissen und Trockenheit der Haut.

Um eine elastische, tragbare Polymerschicht auf der Haut zu einem der vorgenannten Anwendungszwecke bereitzustellen, wird erfindungsgemäß vorgeschlagen, die folgenden Verfahrensschritte auszuführen:
a) topische Applikation des Polymervorläufers auf die zu behandelnde Hautstelle,
b) topische Applikation des Polymerisationskatalysators auf die zu behandelnde Hautstelle,
c) wahlweise topische Applikation eines Photosensibilisators auf die zu behandelnde Hautstelle, und
d) Exposition der zu behandelnden Hautstelle gegenüber Licht,
   wobei die Reihenfolge der Ausführung der Verfahrensschritte variiert werden kann, mit der Maßgabe, dass Verfahrensschritt d) stets der letzte Verfahrensschritt ist.

Vorzugsweise wird eine erfindungsgemäße Zusammensetzung eingesetzt, bei der der Polymervorläufer und der Polymerisationskatalysator in einem vorgefertigten Gemisch vorliegen.

In diesen Fällen vereinfacht sich das Verfahren dadurch, dass nur die folgenden Schritte auszuführen sind:
a) topische Applikation des Gemisches, welches den Polymervorläufer und den Polymerisationskatalysator enthält, auf die zu behandelnde Hautstelle, wahlweise unter gleichzeitiger oder nachfolgender topischer Applikation eines Photosensibilisators auf die zu behandelnde Hautsstelle, und
b) Exposition der zu behandelnden Hautstelle gegenüber Licht.

Unabhängig davon, welches der beiden Verfahren angewandt wird, erfolgt die Exposition der zu behandelnden Hautstelle gegenüber Licht in den für die erfindungsgemäße Zusammensetzung genannten Wellenlängenbereichen für die Polymerisation unter Einwirkung von Licht.

Für Zwecke der ursprünglichen Offenbarung wird darauf hingewiesen, dass sämtliche Merkmale, wie sie sich aus der vorliegenden Beschreibung und den Ansprüchen für einen Fachmann erschließen, auch wenn sie konkret nur im Zusammenhang mit bestimmten weiteren Merkmalen beschrieben wurden, sowohl einzeln als auch in beliebigen Zusammenstellungen mit anderen der hier offenbarten Merkmale oder Merkmalsgruppen kombinierbar sind, soweit dies nicht ausdrücklich ausgeschlossen wurde oder chemische, physikalisch-chemische, kosmetische, pharmakologische oder dermatologische Gegebenheiten derartige Kombinationen unmöglich oder sinnlos machen. Auf die umfassende, explizite Darstellung sämtlicher denkbaren Merkmalskombinationen wird hier nur der Kürze und der Lesbarkeit der Beschreibung wegen verzichtet.

## Patentansprüche

1. Kosmetische oder pharmazeutische Zusammensetzung für die topische Applikation auf der Haut, wobei die Zusammensetzung wenigstens einen Polymervorläufer enthält, **dadurch gekennzeichnet, dass** die Zusammensetzung zusätzlich wenigstens einen Polymerisationskatalysator enthält, in dessen Gegenwart der wenigstens eine Polymervorläufer unter Einwirkung von Licht polymerisiert und wobei die Polymerisation des Polymervorläufers entweder in einem rein lichtinduzierten und -getriebenen Prozess erfolgt oder durch Dualpolymerisation, wobei unter Dualpolymerisation die Initiation des Polymerisationsprozesses durch Licht, mit anschließender chemischer Polymerisation, oder in umgekehrter Reihenfolge zu verstehen ist, wobei der Mechanismus der chemischen Polymerisation unter einer Kondensationsreaktionspolymerisation, einer Additionsreaktionspolymerisation, einer Ringöffnungsreaktionspolymerisation, und/oder einer katalytischen Polymerisation ausgewählt ist.

2. Kosmetische oder pharmazeutische Zusammensetzung nach Anspruch 1, **dadurch gekennzeichnet, dass** der wenigstens eine Polymervorläufer ein Siloxan ist.

3. Kosmetische oder pharmazeutische Zusammensetzung nach einem der Ansprüche 1 und 2, **dadurch gekennzeichnet, dass** der wenigstens eine Polymervorläufer ein polyethermodifiziertes Siloxan ist.

4. Kosmetische oder pharmazeutische Zusammensetzung nach einem der Ansprüche 1 bis 3, **dadurch gekennzeichnet, dass** der wenigstens eine Polymervorläufer ausgewählt ist unter
a) einer Kombination von mindestens einem:
i) Polyorganohydrosiloxan mit folgender Struktur: 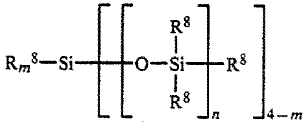 wobei R⁸ unabhängige Reste darstellen, ausgewählt aus einer Gruppe bestehend aus Alkylgruppen, Cycloalkylgruppen, Phenylgruppen, Epoxidgruppen, Isocyanatgruppen, Aminogruppen, Alkoholgruppen Ethergruppen, Estergruppen und Wasserstoff, vorausgesetzt, dass mindestens zwei aber nicht mehr als die Hälfte aller R⁸-Gruppen des Siloxans Wasserstoffe sind und, wobei m 0, 1, 2 oder 3 und n eine Zahl mit einem Wert zwischen 1 und 3000 ist,
und mindestens einem
(ii) Polyorganosiloxan mit folgender Struktur: 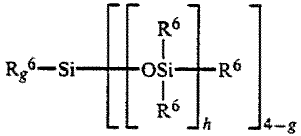 wobei R⁶ unabhängige Reste darstellen, ausgewählt aus einer Gruppe bestehend aus nicht-halogenierten oder halogenierten ethylenylisch ungesättigten Gruppen, nicht halogenierten oder halogenierten Alkylgruppen, nicht halogenierten oder halogenierten Cycloalkylgruppen, Epoxidgruppen, Isocyanatgruppen, Aminogruppen, Alkoholgruppen, Ethergruppen, Estergruppen, Phenylgruppen, vorausgesetzt, dass mindestens 70% aller R⁶-Gruppen Vinylgruppen oder andere Alkenylgruppen enthaltenund wobei h eine Zahl mit einem Wert zwischen 1 und 3000 und g 0, 1, 2, oder 3 ist,
und
b) mindestens einem Polyorganohydrosiloxan mit folgender Struktur: 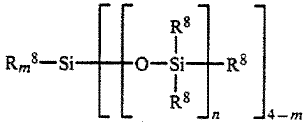 wobei R⁸ unabhängige Reste darstellen, ausgewählt aus einer Gruppe bestehend aus Alkylgruppen, Cycloalkylgruppen, Phenylgruppen, Epoxidgruppen, Isocyanatgruppen, Aminogruppen, Alkoholgruppen, Ethergruppen, Estergruppen, Wasserstoff und nicht-halogenierten oder halogenierten ethylenylisch ungesättigten Gruppen, vorausgesetzt, dass mindestens zwei aber nicht mehr als die Hälfte aller R⁸-Gruppen des Siloxans Wasserstoffe sind und weiterhin vorausgesetzt, dass mindestens zwei der R⁸-Gruppen Vinylgruppen oder Alkenylgruppen enthalten, wobei m 0, 1, 2 oder 3 und n eine Zahl mit einem Wert zwischen 1 und 3000 ist.

5. Kosmetische oder pharmazeutische Zusammensetzung nach einem der Ansprüche 1 bis 4, **dadurch gekennzeichnet, dass** der Polymerisationskatalysator eine Verbindung ist, die unter die allgemeine Formel (I) fällt 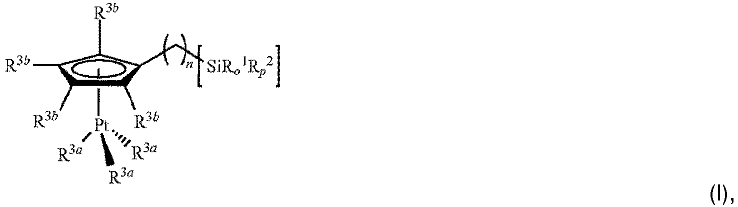 wobei
n=1-8,
o=0-2,
p=1-3 und o+p=3 sind
und die Substituenten R¹ identisch oder unterschiedlich und jeweils, unabhängig voneinander, ein monovalenter, unsubstituierter oder substituierter, linearer, cyclischer oder verzweigter Kohlenwasserstoffrest sind, der aliphatisch gesättigte und ungesättigte oder aromatische Reste enthält und 1-30 Kohlenstoffatome hat, bei denen einzelne Kohlenstoffatome durch O, N, S oder P Atome ersetzt sein können,
die Substituenten R² identisch oder unterschiedlich voneinander, hydrolysierbare funktionelle Gruppen ausgewählt sind aus einer Gruppe bestehend aus:
Carboxy der allgemeinen Formel -O-C(O)R⁴,
Oxim der allgemeinen Formel -O-N=CR⁴₂,
Alkoxy der allgemeinen Formel -OR⁴,
Alkenyloxy der allgemeinen Formel -O-R⁶,
Amid der allgemeinen Formel -NR⁴-C(O)R⁵,
Amin der allgemeinen Formel -NR⁴R⁵,
Aminoxy der allgemeinen Formel -O-NR⁴R⁵,
wobei
die Reste R⁴ identisch oder unterschiedlich und jeweils unabhängig voneinander, H, Alkyl, Aryl, Arylalkyl oder Alkylaryl sind,
die Substituenten R⁵ identisch oder unterschiedlich und jeweils unabhängig voneinander, Alkyl, Aryl, Arylalkyl, oder Alkylaryl sind
R⁶ ein linearer oder verzweigter, aliphatisch ungesättigter Rest ist,
die Substituenten R^{3a} identisch oder unterschiedlich und jeweils unabhängig voneinander, Alkyl, Aryl, Arylalkyl oder Alkylaryl sind, wobei diese 1-30 Kohlenstoffatome aufweisen, wobei die Wasserstoffatome durch -Hal oder -SiR₃³ ersetzt sein können,
die Substituenten R³ identisch oder unterschiedlich und jeweils, unabhängig voneinander, monovalente, unsubstituierte oder substituierte, lineare, cyclische oder verzweigte Kohlenwasserstoffreste sind,
die Substituenten R^{3b} identisch oder unterschiedlich und jeweils unabhängig voneinander Wasserstoff oder ein monovalenter, unsubstituierter oder substituierter, linearer oder verzweigter Kohlenwasserstoffrest sind, wobei dieser einen aliphatisch gesättigten oder ungesättigten oder aromatischen Rest enthält und 1-30 Kohlenstoffatome hat und in dem die einzelnen Kohlenstoffatome durch O, N, S oder P Atome ersetzt sein können.

6. Kosmetische oder pharmazeutische Zusammensetzung nach einem der Ansprüche 1 bis 4, **dadurch gekennzeichnet, dass** der Polymerisationskatalysator eine Verbindung ist, die unter die allgemeine Formel (II) fällt, 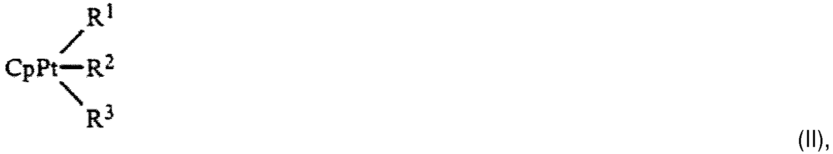 wobei
Cp eine Cyclopentadienylgruppe darstellt, die η-gebunden an das Platinatom vorliegt, wobei die Cyclopentadienylgruppe wahlweise ein- oder mehrfach substituiert ist mit Resten, die nicht bei der Hydrosilylierungsreaktion stören, und
jeder der Reste R¹, R² und R³ eine aliphatische oder aromatische Gruppe mit 1-18 Kohlenstoffatomen darstellt, wobei R¹, R² und R³ σ-gebunden an das Platinatom vorliegen,
und wobei die Zusammensetzung zusätzlich wenigstens einen Photosensibilisator enthält, der dazu geeignet ist, die absorbierte Lichtenergie auf den Polymerisationskatalysator zu übertragen.

7. Kosmetische oder pharmazeutische Zusammensetzung nach Anspruch 6, **dadurch gekennzeichnet, dass** der wenigstens eine Photosensibilisator eine Verbindung ist, die aus einer Gruppe bestehend aus polycyclischen Aromaten mit 2-5 Ringen, Thioxanthonen, 2-Chlorthioxanthonen, 2-Isopropylthioxanthonen, aromatischen Verbindungen mit mindestens einem Ketonchromophor und Kombinationen hiervon ausgewählt ist.

8. Kosmetische oder pharmazeutische Zusammensetzung nach einem der Ansprüche 1 bis 7, **dadurch gekennzeichnet, dass** die Zusammensetzung zusätzlich eine Partikelphase enthält, die aus Partikeln besteht, die ausgewählt sind aus einer Gruppe bestehend aus: Siliconen, Stärke und Stärkederivaten, Cellulosepulver, Polymerpulver aus Polyolefinen, Polycarbonaten, Polyurethanen, Polyamiden, Polyester, Polystyrolen, Polyacrylaten, Polymethacrylaten, (Meth)acrylat- oder (Meth)acrylat-Vinyliden-Copolymeren, Teflon oder Kombinationen hiervon.

9. Kosmetische oder pharmazeutische Zusammensetzung nach einem der Ansprüche 1 bis 8, **dadurch gekennzeichnet, dass** sie dekorative Pigmente und/oder Farbstoffe enthält, ausgewählt aus einer Gruppe bestehend aus Metalleffektpigmenten, Perlglanzpigmenten, Leuchtpigmenten, natürlichen organischen Pigmenten oder synthetisch organischen Pigmenten.

10. Nicht-therapeutische Verwendung einer kosmetischen Zusammensetzung gemäß einem der vorangegangenen Ansprüche zur Hautstraffung, Faltenreduzierung oder zur optischen Kaschierung von Hautfalten, Rissen in der Haut, trockener Haut und/oder von Narbengewebe der Haut und/oder von Hautstellen mit Pigmentstörung.

11. Nicht-therapeutische Verwendung einer kosmetischen Zusammensetzung nach einem der Ansprüche 1 bis 9 zur Formgebung und/oder Fixierung der Haut in einer gewünschten Position und/oder zur Formgebung und/oder Fixierung von Eigenhaar, Fremdhaar oder künstlichem Haar.

12. Nicht-therapeutische Verwendung einer kosmetischen Zusammensetzung nach einem der Ansprüche 1 bis 9 zur Entfernung von Körperhaaren.

13. Nicht-therapeutische Verwendung einer kosmetischen Zusammensetzung nach einem der Ansprüche 10 bis 12 als Flüssigpflaster, vorgefertigtes elastisches Pflaster, chirurgischen Wundverschluss, Blasenpflaster, Wirkstoffpflaster oder Sprühpflaster.

14. Verfahren zur Herstellung einer elastischen, tragbaren Polymerschicht auf der Haut, wobei das Verfahren die folgenden Verfahrensschritte umfasst:
a) topische Applikation eines Polymervorläufers, wie er in einem der vorangegangen Ansprüche definiert ist, auf die zu behandelnde Hautstelle,
b) topische Applikation eines Polymerisationskatalysators, wie er in einem der vorangegangen Ansprüche definiert ist, auf die zu behandelnde Hautstelle,
c) wahlweise topische Applikation eines Photosensibilisators, wie er in einem der vorangegangen Ansprüche definiert ist, auf die zu behandelnde Hautstelle, und
d) Exposition der zu behandelnden Hautstelle gegenüber Licht,
wobei die Reihenfolge der Ausführung der Verfahrensschritte variiert werden kann, mit der Maßgabe, dass Verfahrensschritt d) stets der letzte Verfahrensschritt ist

15. Verfahren nach Anspruch 14, **dadurch gekennzeichnet, dass** die Exposition der zu behandelnden Hautstelle gegenüber Licht gemäß Verfahrensschritt d) durch Bestrahlung mit sichtbarem Licht einer Wellenlänge im Bereich von 400 - 800 nm erfolgt.

## Claims

1. Cosmetic or pharmaceutical composition for topical application to the skin, wherein the composition contains at least one polymer precursor, **characterized in that** the composition additionally contains at least one polymerization catalyst, in the presence of which the at least one polymer precursor polymerizes under the action of light, and wherein the polymerization of the polymer precursor is effected either in a purely light-induced and light-driven process or by dual polymerization, wherein by dual polymerization is meant the initiation of the polymerization process by light followed by chemical polymerization, or in reverse order, wherein the mechanism of the chemical polymerization is selected from a condensation reaction polymerization, an addition reaction polymerization, a ring-opening reaction polymerization and/or a catalytic polymerization.

2. Cosmetic or pharmaceutical composition according to claim 1, **characterized in that** the at least one polymer precursor is a siloxane.

3. Cosmetic or pharmaceutical composition according to one of claims 1 and 2, **characterized in that** the at least one polymer precursor is a polyether-modified siloxane.

4. Cosmetic or pharmaceutical composition according to one of claims 1 to 3, **characterized in that** the at least one polymer precursor is selected from
a) a combination of at least one:
i) polyorganohydrosiloxane with the following structure: 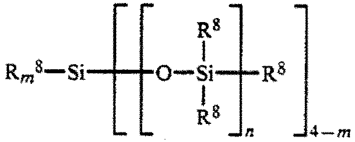 wherein R⁸ represent independent radicals selected from a group consisting of alkyl groups, cycloalkyl groups, phenyl groups, epoxide groups, isocyanate groups, amino groups, alcohol groups, ether groups, ester groups and hydrogen, provided that at least two, but not more than half of all R⁸ groups in the siloxane are hydrogens, and wherein m is 0, 1, 2 or 3 and n is a number with a value between 1 and 3000,
and at least one
ii) polyorganosiloxane with the following structure: 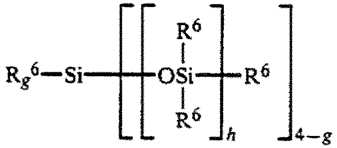 wherein R⁶ represent independent radicals selected from a group consisting of non-halogenated or halogenated ethylenically unsaturated groups, non-halogenated or halogenated alkyl groups, non-halogenated or halogenated cycloalkyl groups, epoxide groups, isocyanate groups, amino groups, alcohol groups, ether groups, ester groups, phenyl groups, provided that at least 70% of all R⁶ groups contain vinyl groups or other alkenyl groups and wherein h is a number with a value between 1 and 3000 and g is 0, 1, 2, or 3,
and
b) at least one polyorganohydrosiloxane with the following structure: 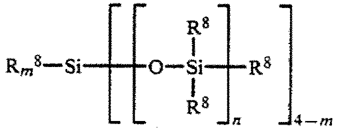 wherein R⁸ represent independent radicals selected from a group consisting of alkyl groups, cycloalkyl groups, phenyl groups, epoxide groups, isocyanate groups, amino groups, alcohol groups, ether groups, ester groups, hydrogen and non-halogenated or halogenated ethylenically unsaturated groups, provided that at least two, but not more than half of all R⁸ groups in the siloxane are hydrogens and furthermore provided that at least two of the R⁸ groups contain vinyl groups or alkenyl groups, wherein m is 0, 1, 2 or 3 and n is a number with a value between 1 and 3000.

5. Cosmetic or pharmaceutical composition according to one of claims 1 to 4, **characterized in that** the polymerization catalyst is a compound which comes under the general formula (I) 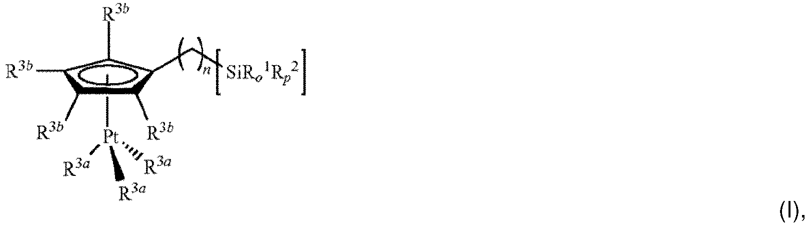 wherein
n=1-8,
o=0-2,
p=1-3 and o+p=3
and the substituents R¹ are identical or different and are in each case, independently of one another, a monovalent, unsubstituted or substituted, linear, cyclic or branched hydrocarbon radical, which contains aliphatically saturated and unsaturated or aromatic radicals and has 1-30 carbon atoms in which individual carbon atoms can be replaced by O, N, S or P atoms,
the substituents R² are hydrolyzable functional groups, identical or different from one another, selected from a group consisting of:
carboxy of the general formula -O-C(O)R⁴,
oxime of the general formula -O-N=CR⁴₂,
alkoxy of the general formula -OR⁴,
alkenyloxy of the general formula -O-R⁶,
amide of the general formula -NR⁴-C(O)R⁵,
amine of the general formula -NR⁴R⁵,
amineoxy of the general formula -O-NR⁴R⁵,
wherein
the radicals R⁴ are identical or different and are in each case, independently of one another, H, alkyl, aryl, arylalkyl or alkylaryl,
the substituents R⁵ are identical or different and are in each case, independently of one another, alkyl, aryl, arylalkyl, or alkylaryl,
R⁶ is a linear or branched, aliphatically unsaturated radical,
the substituents R^{3a} are identical or different and are in each case, independently of one another, alkyl, aryl, arylalkyl or alkylaryl, wherein these have 1-30 carbon atoms, wherein the hydrogen atoms can be replaced by -Hal or -SiR₃³,
the substituents R³ are identical or different and are in each case, independently of one another, monovalent, unsubstituted or substituted, linear, cyclic or branched hydrocarbon radicals,
the substituents R^{3b} are identical or different and are in each case, independently of one another, hydrogen or a monovalent, unsubstituted or substituted, linear or branched hydrocarbon radical, wherein this contains an aliphatically saturated or unsaturated or aromatic radical and has 1-30 carbon atoms and in which the individual carbon atoms can be replaced by O, N, S or P atoms.

6. Cosmetic or pharmaceutical composition according to one of claims 1 to 4, **characterized in that** the polymerization catalyst is a compound which comes under the general formula (II), 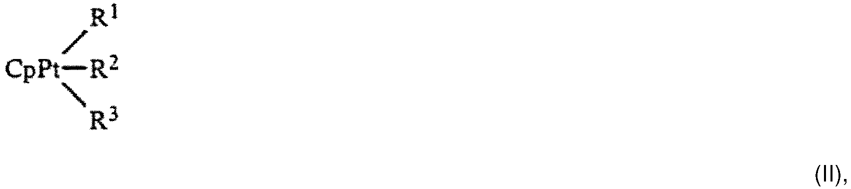 wherein
Cp represents a cyclopentadienyl group which is η-bonded to the platinum atom, wherein the cyclopentadienyl group is optionally mono- or polysubstituted by radicals which do not interfere with the hydrosilylation reaction, and
each of the radicals R¹, R² and R³ represents an aliphatic or aromatic group with 1-18 carbon atoms, wherein R¹, R² and R³ are σ-bonded to the platinum atom,
and wherein the composition additionally contains at least one photosensitizer which is suitable for transferring the absorbed light energy to the polymerization catalyst.

7. Cosmetic or pharmaceutical composition according to claim 6, **characterized in that** the at least one photosensitizer is a compound which is selected from a group consisting of polycyclic aromatics with 2-5 rings, thioxanthones, 2-chlorothioxanthones, 2-isopropylthioxanthones, aromatic compounds with at least one ketone chromophore and combinations thereof.

8. Cosmetic or pharmaceutical composition according to one of claims 1 to 7, **characterized in that** the composition additionally contains a particle phase which consists of particles which are selected from a group consisting of: silicones, starch and starch derivatives, cellulose powders, polymer powders comprising polyolefins, polycarbonates, polyurethanes, polyamides, polyesters, polystyrenes, polyacrylates, polymethacrylates, (meth)acrylate or (meth)acrylate-vinylidene copolymers, Teflon or combinations thereof.

9. Cosmetic or pharmaceutical composition according to one of claims 1 to 8, **characterized in that** it contains decorative pigments and/or dyes selected from a group consisting of metal-effect pigments, pearlescent pigments, luminescent pigments, natural organic pigments or synthetic organic pigments.

10. Non-therapeutic use of a cosmetic composition according to one of the preceding claims for skin tautening, wrinkle reduction or for visually concealing skin wrinkles, cracks in the skin, dry skin and/or scar tissue of the skin and/or skin areas with pigmentary abnormality.

11. Non-therapeutic use of a cosmetic composition according to one of claims 1 to 9 for shaping and/or fixing the skin in a desired position and/or for shaping and/or fixing one's own hair, someone else's hair or artificial hair.

12. Non-therapeutic use of a cosmetic composition according to one of claims 1 to 9 for the removal of body hairs.

13. Non-therapeutic use of a cosmetic composition according to one of claims 10 to 12 as a liquid plaster, ready-made elastic plaster, surgical wound closure, blister plaster, active-ingredient plaster or spray-on plaster.

14. Process for the production of an elastic, wearable polymer layer on the skin, wherein the process comprises the following process steps:
a) topical application of a polymer precursor, as defined in one of the preceding claims, to the skin area to be treated,
b) topical application of a polymerization catalyst, as defined in one of the preceding claims, to the skin area to be treated,
c) optionally topical application of a photosensitizer, as defined in one of the preceding claims, to the skin area to be treated, and
d) exposure of the skin area to be treated to light,
wherein the sequence in which the process steps are carried out can be varied, with the proviso that process step d) is always the last process step.

15. Process according to claim 14, **characterized in that** the exposure of the skin area to be treated to light according to process step d) is effected by irradiation with visible light of a wavelength in the range of 400 - 800 nm.

## Revendications

1. Composition cosmétique ou pharmaceutique pour application topique sur la peau, la composition contenant au moins un précurseur de polymères, **caractérisée en ce que** la composition contient en outre au moins un catalyseur de polymérisation, en présence duquel le précurseur de polymères, au moins au nombre de un, est polymérisé sous l'action de la lumière, et la polymérisation du précurseur de polymères est réalisée soit dans un procédé induit et actionné par la lumière, soit par polymérisation double, une polymérisation double s'entendant comme étant l'amorçage du processus de polymérisation par la lumière, suivi d'une polymérisation chimique, ou dans l'ordre inverse, le mécanisme de la polymérisation chimique étant choisi parmi une polymérisation par réaction de condensation, une polymérisation par réaction d'addition, une polymérisation par ouverture des cycles, et/ou une polymérisation catalytique.

2. Composition cosmétique ou pharmaceutique selon la revendication 1, **caractérisée en ce que** le précurseur de polymères, au moins au nombre de un, est un siloxane.

3. Composition cosmétique ou pharmaceutique selon l'une des revendications 1 et 2, **caractérisée en ce que** le précurseur de polymères, au moins au nombre de un, est un siloxane modifié par un polyéther.

4. Composition cosmétique ou pharmaceutique selon l'une des revendications 1 à 3, **caractérisée en ce que** le précurseur de polymères, au moins au nombre de un, est choisi parmi
a) une combinaison d'au moins un :
i) polyorganohydrosiloxane ayant la structure suivante : 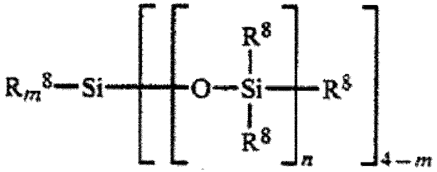 dans laquelle les radicaux R⁸ représentent des radicaux indépendants, choisis dans un groupe composé de groupes alkyle, de groupes cycloalkyle, de groupes phényle, de groupes époxyde, de groupes isocyanate, de groupes amino, de groupes alcool, de groupes éther, de groupes ester et de l'hydrogène, à la condition qu'au moins deux, mais pas plus de la moitié de tous les groupes R⁸ du siloxane représentent des atomes d'hydrogène, m représentant 0, 1, 2 ou 3, et n représentant un nombre ayant une valeur comprise entre 1 et 3000, et d'au moins un
(ii) polyorganosiloxane ayant la structure suivante : 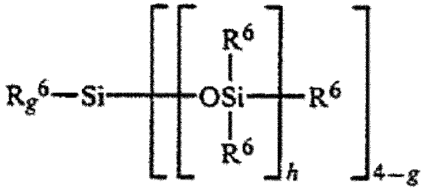 dans laquelle les radicaux R⁶ représentent des radicaux indépendants, choisis dans un groupe composé des groupes à insaturation éthylénique non halogénés ou halogénés, des groupes alkyle non halogénés ou halogénés, des groupes cycloalkyle non halogénés ou halogénés, des groupes époxyde, des groupes isocyanate, des groupes amino, des groupes alcool, des groupes éther, des groupes ester, des groupes phényle, à la condition qu'au moins 70 % de tous les groupes R⁶ contiennent des groupes vinyle ou d'autres groupes alcényle, et h représentant un nombre ayant une valeur comprise entre 1 et 3000, et g représentant 0, 1, 2 ou 3,
et
b) d'au moins un polyorganohydrosiloxane ayant la structure suivante : 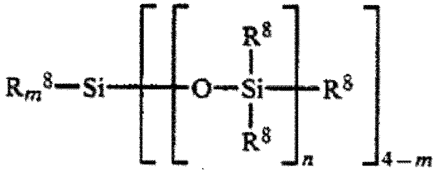 dans laquelle les radicaux R⁸ représentent des radicaux indépendants, choisis dans un groupe composé des groupes alkyle, des groupes cycloalkyle, des groupes phényle, des groupes époxyde, des groupes isocyanate, des groupes amino, des groupes alcool, des groupes éther, des groupes ester, de l'hydrogène et des groupes à insaturation éthylénique non halogénés ou halogénés, à la condition qu'au moins deux, mais pas plus de la moitié de tous les groupes R⁸ du siloxane représentent des atomes d'hydrogène, et à la condition en outre qu'au moins deux des groupes R⁸ contiennent des groupes vinyle ou des groupes alcényle, m représentant 0, 1, 2 ou 3 et n représentant un nombre ayant une valeur comprise entre 1 et 3000.

5. Composition cosmétique ou pharmaceutique selon l'une des revendications 1 à 4, **caractérisée en ce que** le catalyseur de polymérisation est un composé qui entre dans le cadre de la formule générale (I) 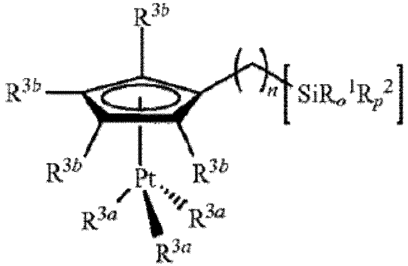 dans laquelle
n = 1 à 8,
o = 0 à 2,
p = 1 à 3 et o+p = 3
et les substituants R¹ sont identiques ou différents et représentent chacun, indépendamment les uns des autres, un radical hydrocarbure monovalent, non substitué ou substitué, linéaire, cyclique ou ramifié, qui contient des radicaux à saturation ou insaturation aliphatique, ou aromatiques, et contient 1 à 30 atomes de carbone, dans lesquels des atomes de carbone individuels peuvent être remplacés par des atomes 0, N, S ou P,
les substituants R² sont identiques ou différents les uns des autres et représentent des groupes fonctionnels hydrolysables choisis dans un groupe composé de :
un carboxy de formule générale -O-C(O)R⁴,
une oxime de formule générale -O-N=CR⁴₂,
un alcoxy de formule générale -OR⁴,
un alcényloxy de formule générale -O-R⁶,
un amide de formule générale -NR⁴-C(O)R⁵,
une amine de formule générale -NR⁴R⁵,
un aminoxy de formule générale -O-NR⁴R⁵,
où
les radicaux R⁴ sont identiques ou différents et représentant chacun, indépendamment des autres, H, un alkyle, un aryle, un arylalkyle ou un alkylaryle,
les substituants R⁵ sont identiques ou différents et représentent chacun indépendamment des autres un alkyle, un aryle, un arylalkyle ou un alkylaryle,
R⁶ représente un radical linéaire ou ramifié à insaturation aliphatique,
les substituants R^{3a} sont identiques ou différents et représentent chacun indépendamment des autres un alkyle, un aryle, un arylalkyle ou un alkylaryle, ceux-ci comportant 1 à 30 atomes de carbone, les atomes de carbone pouvant être remplacés par -Hal ou -SiR₃³,
les substituants R³ sont identiques ou différents et représentent chacun indépendamment des autres des radicaux hydrocarbure monovalents, substitués ou non substitués, linéaires, cycliques ou ramifiés,
les substituants R^{3b} sont identiques ou différents et représentent chacun indépendamment des autres un hydrogène ou un radical hydrocarbure monovalent, non substitué ou substitué, linéaire ou ramifié, ceux-ci contenant un radical à saturation ou insaturation aliphatique, ou aromatique, et comportant 1 à 30 atomes de carbone, les atomes de carbone individuels pouvant être remplacés par des atomes O, N, S ou P.

6. Composition cosmétique ou pharmaceutique selon l'une des revendications 1 à 4, **caractérisée en ce que** le catalyseur de polymérisation est un composé qui entre dans le cadre de la formule générale (II), 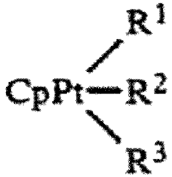 dans laquelle
Cp représente un groupe cyclopentadiényle, qui est présent lié par une liaison η à l'atome de platine, le groupe cyclopentadiényle étant une ou plusieurs fois substitué par des radicaux qui ne provoquent aucune perturbation lors de la réaction d'hydrosilylation, et
chacun des radicaux R¹, R² et R³ représente un groupe aliphatique ou aromatique ayant 1 à 18 atomes de carbone, R¹, R² et R³ étant liés par une liaison σ à l'atome de platine,
et la composition contenant en outre au moins un photosensibilisateur qui est apte à transmettre l'énergie lumineuse absorbée au catalyseur de polymérisation.

7. Composition cosmétique ou pharmaceutique selon la revendication 6, **caractérisée en ce que** le photosensibilisateur, au moins au nombre de un, est un composé qui est choisi dans un groupe composé des composés aromatiques polycycliques ayant 2 à 5 cycles, des thioxanthones, des 2-chlorothioxanthones, des 2-isopropylthioxanthones, des composés aromatiques ayant au moins un chromophore de type cétone et de combinaisons de ceux-ci.

8. Composition cosmétique ou pharmaceutique selon l'une des revendications 1 à 7, **caractérisée en ce que** la composition contient en outre une phase particulaire qui est constituée de particules qui sont choisies dans un groupe composé de : silicones, amidon et dérivés de l'amidon, poudre de cellulose, poudre polymère constituée de polyoléfines, polycarbonates, polyuréthannes, polyamides, polyesters, polystyrènes, polyacrylates, polyméthacrylates, copolymères de (méth)acrylate- ou (méth)acrylate-vinylidènes, Téflon ou combinaisons de ceux-ci.

9. Composition cosmétique ou pharmaceutique selon l'une des revendications 1 à 8, **caractérisée en ce qu'**elle contient des pigments et/ou des colorants décoratifs, choisis dans un groupe composé de pigments à effets métalliques, de pigments nacrés, de pigments luminescents, de pigments organiques naturels et de pigments organiques synthétiques.

10. Utilisation non thérapeutique d'une composition cosmétique selon l'une des revendications précédentes pour le raffermissement de la peau, pour la réduction des rides ou pour le floutage optique des rides cutanées, des craquelures de la peau, de la peau sèche et/ou des tissus cicatriciels de la peau et/ou des zones de la peau présentant un trouble de la pigmentation.

11. Utilisation non thérapeutique d'une composition cosmétique selon l'une des revendications 1 à 9 pour façonner et/ou fixer la peau dans une position souhaitée et/ou pour former et/ou fixer les cheveux naturels, les cheveux étrangers ou les cheveux artificiels.

12. Utilisation non thérapeutique d'une composition cosmétique selon l'une des revendications 1 à 9 pour éliminer les poils corporels.

13. Utilisation non thérapeutique d'une composition cosmétique selon l'une des revendications 10 à 12 en tant qu'emplâtre liquide, pansement élastique préfabriqué, fermeture chirurgicale des plaies, pansement pour ampoules, pansement à libération de principes actifs ou pansement en spray.

14. Procédé de fabrication d'une couche polymère élastique, pouvant être portée sur la peau, le procédé comprenant les étapes suivantes :
a) application topique d'un précurseur de polymères tel que défini dans l'une des revendications précédentes sur la zone de la peau à traiter,
b) application topique d'un catalyseur de polymérisation tel que défini dans l'une des revendications précédentes sur la zone de la peau à traiter,
c) éventuellement, application topique d'un photosensibilisateur tel que défini dans l'une des revendications précédentes sur la zone de la peau à traiter, et
d) exposition à la lumière de la zone de la peau à traiter,
dans lequel on peut faire varier l'ordre d'exécution des étapes, à la condition que l'étape d) soit toujours la dernière étape du procédé.

15. Procédé selon la revendication 14, **caractérisé en ce que** l'exposition à la lumière de la zone de la peau à traiter selon l'étape de procédé d) s'effectue par irradiation avec une lumière visible ayant une longueur d'onde comprise dans la plage de 400 à 800 nm.
